Europäisches Patentamt

European Patent Office

Office européen des brevets

(19)

(11) Veröffentlichungsnummer: **0 217 146**
**B1**

(12) **EUROPÄISCHE PATENTSCHRIFT**

(45) Veröffentlichungstag der Patentschrift: **12.09.90**

(21) Anmeldenummer: **86111974.1**

(22) Anmeldetag: **29.08.86**

(51) Int. Cl.⁵: **A 61 K 31/57,** A 61 K 47/00, A 61 K 9/10

(54) **Hydrocortisondiester enthaltende O/W-Creme.**

(30) Priorität: **28.09.85 DE 3534743**

(43) Veröffentlichungstag der Anmeldung:
**08.04.87 Patentblatt 87/15**

(45) Bekanntmachung des Hinweises auf die
Patenterteilung:
**12.09.90 Patentblatt 90/37**

(84) Benannte Vertragsstaaten:
**AT BE CH DE FR GB IT LI LU NL SE**

(56) Entgegenhaltungen:
EP-A-0 098 566
EP-A-0 131 821
DE-A-2 851 544
FR-A-2 539 991
FR-A-2 539 992

CHEMICAL ABSTRACTS, Band 103, 1985, Seite
358, Zusammenfassung Nr. 220828m,
Columbus, Ohio, US; & JP-A-60 156 608
(TAISHO PHARMACEUTICAL CO., LTD) 16-08-
1985

(73) Patentinhaber: **Beiersdorf Aktiengesellschaft**
**Unnastrasse 48**
**D-2000 Hamburg 20 (DE)**

(72) Erfinder: **Sattler, Henning, Dr.**
**Jahnring 15**
**D-2000 Hamburg 60 (DE)**
Erfinder: **Asmussen, Bodo, Dr.**
**Dorotheenweg 1a**
**D-2071 Ammersbek (DE)**

EP 0 217 146 B1

**Beschreibung**

Die vorliegende Erfindung bezieht sich auf eine neue O/W-Creme, die Hydrocortison-17-propionat-21-acetat enthält.

Aus der DE—OS 3 402 877 ist bereits eine Ölige Salbe bzw. Fettsalbe mit dem Wirkstoff Hydrocortison-17-butyrat-21-propionat bekannt.

Salben, die keinen oder nur einen sehr geringen Wassergehalt aufweisen, bilden keine Emulsionen und sind somit keine Cremes. Hinsichtlich der Wirkstoffresorption sind solche Zubereitungen nicht immer befriedigend. Auch ist ihr Anwendung mit Unannehmlichkeiten verbunden.

Weiterhin ist aus der DE—A—34 02 880 eine O/W-Creme mit dem Wirkstoff Hydrocortison-17-butyrat-21-propionat bekannt. Aus FR—A—2 539 992 ist eine ähnliche O/W-Creme bekannt, wobei der Emulgator ein Sorbitan-ester ist.

Die beschriebene Cremegrundlage hat sich jedoch nicht immer, insbesondere hinsichtlich der Lagerungsstabilität, beziehungsweise der Beständigkeit des Wirkstoffgehalts, als zufriedenstellend erwiesen. Dies ist in besonderem Maße dann der Fall, wenn andere Hydrocortisondiester als der dort beschriebene Diester mit der angegebenen Grundlage verwendet werden.

Aufgabe der Erfindung ist es, eine Hydrocortison-17-propionat-21-acetat enthaltende O/W-Creme bereitzustellen, die eine gute Lagerungsstabilität und eine hohe Absorption des Wirkstoffes durch die Haut gewährleistet.

Diese Aufgabe wird gelöst durch eine O/W-Creme, die dadurch gekennzeichnet ist, daß sie

0,01—0,5 Gew.% Hydrocortison-17-propionat-21- acetat
5—20 Gew.% O/W-Emulgator auf Basis von Polyoxyethylen-Fettsäureestern und Fettalkoholen
0—10 Gew.% Stearylalkohol
1—50 Gew.% weißes Vaselin
0—5 Gew.% Benzylalkohol und
20—80 Gew.% Wasser

enthält.

Vorzugsweise enthält die O/W-Creme

0,025—0,2 Gew.% Hydrocortison-17-propionat-21-acetat
7,5—15 Gew.% O/W-Emulgator auf Basis von Polyoxyethylen-Fettsäureestern und Fettalkoholen
0,1—5 Gew.% Stearylalkohol
5—25 Gew.% weißes Vaselin
0,1—3 Gew.% Benzylalkohol und
30—70 Gew.% Wasser,

insbesondere aber:

0,13 Gew.% Hydrocortison-17-propionat-21-acetat
12,5 Gew.% O/W-Emulgator auf Basis von Polyoxyethylen-Fettsäureestern und Fettalkoholen
3,5 Gew.% Stearylalkohol
15 Gew.% weißes Vaselin
2,2 Gew.% Benzylalkohol
66,67 Gew.% Wasser.

Alle Mengenangaben, Anteile und Prozentanteile sind auf das Gewicht bezogen.

Die erfindungsgemäßen Zusammensetzungen zeichnen sich durch eine besonders hohe Lagerungsstabilität und gute Wirksamkeit aus. Auch nach mehrjährigere Lagerung ist praktisch keine Abnahme des Wirkstoffgehalts meßbar.

Vorzugsweise wird der Fettalkoholanteil des O/W-Emulgators von höheren Fettalkoholen gebildet. In der Literatur beschriebene Emulgatoren (Lexikon der Hilfsstoffe für Pharmazie, Kosmetik und angrenzende Gebiete, H. P. Fiedler, 1971 und 1981, Editio Cantor Aulendorf) sind unter dem Handelsnamen Crodawachs® und Polawachs® im Handel erhältlich (Firma Croda, DE). Bevorzugt wird Crodawachs® GP200.

Vorzugsweise enthält die Creme Stearylalkohol und Benzylalkohol als Konservierungsmittel in den angegebenen Mengen.

Weiterhin können Zusatzstoffe wie Glycerin, Propylenglykol, Isopropylfettsäureester wie Isopropylmyristat und Isopropylpalmitat, Wachse, beispielsweise Kohlenwasserstoffwachse wie Ozokerit sowie Bienenwachs und Walrat und deren Substitute in kleineren Anteilen enthalten sein, ebenso wie Mittel zur pH-Regulierung, die aber im allgemeinen in der erfindungsgemäßen Creme nicht erforderlich sind.

Mit der neuen erfindungsgemäen Creme wird eine Hydrocortison-17-propionat-21-acetat enthaltende O/W-Creme geschaffen, die sich durch gute Wirksamkeit und hohe Lagerstabilität auszeichnet.

Zur Herstellung der Creme werden die Bestandteile der Fettphase, Vaselin, Emulgator und Stearylalkohol geschmolzen und auf 60—80°C gebracht. Wasser wird ebenfalls auf 60—80°C erhitzt und mit der Fettphase vermischt. Bei etwa 60°C wird der Wirkstoff Hydrocortison-17-propionat-21-acetat zugegeben. Unter Rühren läßt man die Masse auf etwa 40°C abkühlen und gibt das Konservierungsmittel Benzylalkohol zu. Unter weiterem Rühren läßt man die Masse abkühlen und erstarren.

Aus der erfindungsgemäßen Creme, die eine hervorrangende Lagerungsstabilität aufweist, wird der Wirkstoff sehr gut absorbiert. Sie wird zur Behandlung von Ekzemen, Dermatitis, Psoriasis sowie Entzündungen verwendet.

Zur Heilung oder Behandlung dieser Erkrankungen kann die erfindungsgemäße Creme topisch auf die geschädigten Stellen aufgebracht werden. Die aufgebrachte Menge der Creme variiert entsprechend der Konzentration des Wirkstoffs der Creme. Im allgemeinen wird eine geeignete Menge auf die geschädigte Stelle mehrmals täglich je nach Schwere der zu behandelnden Erkrankung aufgebracht.

Die folgenden Beispiele dienen zur Erläuterung der Erfindung:

### Beispiel 1

Mit den angegebenen Bestandteilen wird eine O/W-Creme hergestellt:

| | |
|---|---|
| Hydrocortison-17-propionat-21-acetat | 0,127 g |
| O/W-Emulgator auf Basis von Polyoxyethylen-Fettsäureestern und Fettalkoholen (Crodawachs® GP 200) | 12,500 g |
| Stearylalkohol | 3,500 g |
| weißes Vaselin, | 15,000 g |
| Benzylalkohol | 2,200 g |
| gereinigtes Wasser | 66,673 g |
| | 100,000 g |

Vaselin, Stearylalkohol und Emulgator werden auf 75°C erhitzt und mit Wasser gleicher Temperatur vermischt. Nach Abkühlen auf etwa 60°C wird das Hydrocortison-17-propionat-21-acetat zugegeben und die Masse bis zum Erkalten gerührt, wobei bei etwa 40°C der Benzylalkohol zugefügt wird.

### Beispiel 2

Mit den angegebenen Bestandteilen wird eine O/W-Creme hergestellt:

| | |
|---|---|
| Hydrocortison-17-propionat-21-acetat | 0,100 g |
| O/W-Emulgator auf Basis von Polyoxyethylen-Fettsäureestern und Fettalkoholen (Crodawachs® GP 200) | 8,0 g |
| Stearylalkohol | 8,0 g |
| weißes Vaselin, | 20,0 g |
| Benzylalkohol | 2,200 g |
| gereinigtes Wasser | 61,7 g |
| | 100,000 g |

Vaselin, Stearylalkohol und Emulgator werden auf 75°C erhitzt und mit Wasser gleicher Temperatur vermischt. Nach Abkühlen auf etwa 60°C wird das Hydrocortison-17-propionat-21-acetat zugegeben und die Masse bis zum Erkalten gerührt, wobei bei etwa 40°C der Benzylalkohol zugefügt wird.

Beispiel 3

Mit den angegebenen Bestandteilen wird eine O/W-Creme hergestellt:

| | |
|---|---|
| Hydrocortison-17-propionat-21-acetat | 0,05 g |
| O/W-Emulgator auf Basis von Polyoxyethylen-Fettsäureestern und Fettalkoholen (Crodawachs® GP 200) | 15,0 g |
| weißes Vaselin | 15,000 g |
| Benzylalkohol | 1,5 g |
| gereinigtes Wasser | 68,45 g |
| | 100,000 g |

Vaselin und Emulgator werden auf 75°C erhitzt und mit Wasser gleicher Temperatur vermischt. Nach Abkühlen auf etwa 60°C wird das Hydrocortison-17-propionat-21-acetat zugegeben und die Masse bis zum Erkalten gerührt, wobei bei etwa 40°C der Benzylalkohol zugefügt wird.

**Patentansprüche für die Vertragsstaaten: BE CH DE FR GB IT LI LU NL SE**

1. Hydrocortisondiester enthaltende Creme, dadurch gekennzeichnet, daß sie
0,01—0,5 Gew.% Hydrocortison-17-propionat-21-acetat
5—20 Gew.% O/W-Emulgator auf Basis von Polyoxyethylen-Fettsäureestern und Fettalkoholen
0—10 Gew.% Stearylalkohol
1—50 Gew.% weißes Vaselin
0—5 Gew.% Benzylalkohol und
20—80 Gew.% Wasser
enthält.

2. Creme gemäß Anspruch 1, dadurch gekennzeichnet, daß sie aus
0,13 Gew.% Hydrocortison-17-propionat-21-acetat
12,5 Gew.% O/W-Emulgator auf Basis von Polyoxyethylen-Fettsäureestern und Fettalkoholen
3,5 Gew.% Stearylalkohol
15 Gew.% weißem Vaselin
2,2 Gew.% Benzylalkohol und
66,67 Gew.% Wasser
besteht.

3. Creme gemäß Anspruch 1 zur Behandlung von Entzündungen, Psoriasis und Ekzemen.

4. Verfahren zur Herstellung der Creme gemäß Anspruch 1, dadurch kennzeichnet, daß man
5—20 Gew.% O/W-Emulgator auf Basis von Polyoxyethylen-Fettsäureestern und Fettalkoholen
0—10 Gew.% Stearylalkohol und
1—50 Gew.% weißes Vaselin
mit auf 60—80°C erhitztem Wasser (20—80 Gew.%) vermischt und bei etwa 60°C 0,01—0,5 Gew.% Hydrocortison-17-propionat-21-acetat und bei etwa 40°C 0—5 Gew.% Benzylalkohol zugibt.

**Patentansprüche für den Verstragsstaat: AT**

1. Verfahren zur Herstellung einer Hydrocortisondiester enthaltende Creme, dadurch gekennzeichnet, daß sie
0,01—0,5 Gew.% Hydrocortison-17-propionat-21-acetat
5—20 Gew.% O/W-Emulgator auf Basis von Polyoxyethylen-Fettsäureestern und Fettalkoholen
0—10 Gew.% Stearylalkohol
1—50 Gew.% weißes Vaselin
0—5 Gew.% Benzylalkohol und
20—80 Gew.% Wasser
zu einer Creme verarbeitet.

2. Creme gemäß Anspruch 1, dadurch gekennzeichnet, daß sie aus
0,13 Gew.% Hydrocortison-17-propionat-21-acetat
12,5 Gew.% /W-Emulgator auf Basis von Polyoxyethylen-Fettsäureestern und Fettalkoholen
3,5 Gew.% Stearylalkohol
15 Gew.% weißes Vaselin
2,2 Gew.% Benzylalkohol und

6,67 Gew.% Wasser

3. Verfahren zur Herstellung einer Creme zur Behandlung von Entzündungen, Psoriasis und Ekzemen gemäß Anspruch 1.

4. Verfahren zur Herstellung der Creme gemäß Anspruch 1, dadurch kennzeichnet, daß man

5—20 Gew.% O/W-Emulgator auf Basis von Polyoxyethylen-Fettsäureestern und Fettalkoholen
0—10 Gew.% Stearylalkohol und
1—50 Gew.% weißes Vaselin mit auf 60—80°C erhitztem Wasser (20—80 Gew.%) vermischt und bei etwa 60°C 0,01—0,5 Gew.% Hydrocortison-17-propionat-21-acetat und bei etwa 40°C 0—5 Gew.% Benzylalkohol zugibt.

**Revendications pour les Etats contractants: BE CH DE FR GB IT LI LU NL SE**

1. Crème contenant un diester d'hydrocortisone, caractérisée en ce qu'elle conteint
0,01—0,5% en poids de 17-propionate 21-acétate d'hydrocortisone,
5—20% en poids d'émulsifiant huile dans l'eau à base d'esters d'acides gras polyéthoxylés et d'alcools gras,
0—10% en poids d'alcool stéarylique,
1—50% en poids de vaseline blanche,
0—5% en poids d'alcool benzylique, et
20—80% en poids d'eau.

2. Crème selon la revendication 1, caractérisée en ce qu'elle est constituée par
0,13% en poids de 17-propionate 21-acétate d'hydrocortisone,
12,5% en poids d'émulsifiant huile dans l'eau à base d'esters d'acides gras polyéthoxylés et d'alcools gras,
3,5% en poids d'alcool stéarylique,
15% en poids de vaseline blanche,
2,2% en poids d'alcool benzylique, et
66,67% en poids d'eau.

3. Crème selon la revendication 1, pour le traitement d'inflammations, de psoriasis et d'eczémas.

4. Procédé de préparation de la crème selon la revendication 1, caractérisé en ce que l'on mélange avec de l'eau (20—80% en poids), qui a été chauffée à 60—80°C,
5—20% en poids d'émulsifiant huile dans l'eau à base d'esters d'acides gras polyéthoxylés et d'alcools gras,
0—10% en poids d'alcool stéarylique,
1—50% en poids de vaseline blanche,
et que l'on ajoute 0,01—0,5% en poids de 17-propionate 21-acétate d'hydrocortisone à environ 60°C, et que l'on ajoute 0—5% en poids d'alcool benzylique à environ 40°C.

**Revendications pour l'Etat contractant: AT**

1. Procédé de préparation d'une crème contenant un diester d'hydrocortisone, caractérisé en ce que l'on traite, pour obtenir une crème,
0,01—0,5% en poids de 17-propionate 21-acétate d'hydrocortisone,
5—20% en poids d'émulsifiant huile dans l'eau à base d'esters d'acides gras polyéthoxylés et d'alcools gras,
0—10% en poids d'alcool stéarylique,
1—50% en poids de vaseline blanche,
0—5% en poids d'alcool benzylique, et
20—80% en poids d'eau.

2. Procédé de préparation d'une crème selon la revendication 1, caractérisé en ce qu'elle est constituée par
0,13% en poids de 17-propionate 21-acétate d'hydrocortisone,
12,5% en poids d'émulsifiant huile dans l'eau à base d'esters d'acides gras polyéthoxylés et d'alcools gras,
3,5% en poids d'alcool stéarylique,
15% en poids de vaseline blanche,
2,2% en poids d'alcool benzylique, et
66,67% en poids d'eau.

3. Procédé de préparation d'une crème pour le traitement d'inflammations, de psoriasis et d'eczémas selon la revendication 1.

4. Procédé de préparation de la crème selon la revendication 1, caractérisé en ce que l'on mélange avec de l'eau (20—80% en poids), qui a été chauffée à 60—80°C,
5—20% en poids d'émulsifiant huile dans l'eau à base d'esters d'acides gras polyéthoxylés et d'alcools gras,
0—10% en poids d'alcool stéarylique,

5

1—50% en poids de vaseline blanche,
et que l'on ajoute 0,01—0,5% en poids de 17-propionate 21-acétate d'hydrocortisone à environ 60°C, et que l'on ajoute 0—5% en poids d'alcool benzylique à environ 40°C.

**Claims for the Contracting States: BE CH DE FR GB IT LI LU NL SE**

1. Cream containing hydrocortisone diester, characterized in that it contains
0,01—0,5% by weight of hydrocortisone 17-propionate 21-acetate
5—20%
by weight of O/W emulsifier based on polyoxyethylene fatty acid esters and fatty alcohols
—10%
by weight of stearyl alcohol
1—50% by weight of white vaseline
0—5% by weight of benzyl alcohol and
20—80% by weight of water.
2. Cream according to Claim 1, characterized in that is consists of
0,13% by weight of hydrocortisone 17-propionate 21-acetate
12.5% by weight of O/W emulsifier based on polyoxyethylene fatty acid esters and fatty alcohols
3.5% by weight of stearyl alcohol
15% by weight of white vaseline
2.2% by weight of benzyl alcohol and
66.67% by weight of water.
3. Cream according to Claim 1 for the treatment of inflammations, psoriasis and eczemas.
4. Process for the preparation of the cream according to Claim 1, characterized in that
5—20% by weight of O/W emulsifier based on polyoxyethylene fatty acid esters and fatty alcohols
0—10% by weight of stearyl alcohol and
1—50% by weight of white vaseline
are mixed with water (20—80% by weight) which has been heated to 60—80°C, and 0.01—0.5% by weight of hydrocortisone 17-propionate 21-acetate is added at about 60°C, and 0—5% by weight of benzyl alcohol is added at about 40°C.

**Claims for the Contracting State: AT**

1. Process for the preparation of a cream containing hydrocortisone diester, characterized in that
0,01—0,5% by weight of hydrocortisone 17-propionate 21-acetate
5—20% by weight of O/W emulsifier based on polyoxyethylene fatty acid esters and fatty alcohols
0—10% by weight of stearyl alcohol
1—50% by weight of white vaseline
0—5% by weight of benzyl alcohol and
20—80% by weight of water are processed to give a cream.
2. Process for the preparation of a cream according to Claim 1, characterized in that it consists of
0,13% by weight of hydrocortisone 17-propionate 21-acetate
12.5% by weight of O/W emulsifier based on polyoxyethylene fatty acid esters and fatty alcohols
3.5% by weight of stearyl alcohol
15% by weight of white vaseline
2.2% by weight of benzyl alcohol and
66.67% by weight of water.
3. Process for the preparation of a cream for the treatment of inflammations, psoriasis and eczemas according to Claim 1
4. Process for the preparation of the cream according to Claim 1, characterized in that
5—20% by weight of O/W emulsifier based on polyoxyethylene fatty acid esters and fatty alcohols
0—10% by weight of stearyl alcohol and
1—50% by weight of white vaseline
are mixed with water (20—80 by weight) which has been heated to 60—80°C, and 0.01—0.5% by weight of hydrocortisone 17-propionate 21-acetate is added at about 60°C, and 0—5% by weight of benzyl alcohol is added at about 40°C.